# FASCICULE DE BREVET EUROPEEN

(11) **EP 1 152 023 B1**
(45) Date de publication et mention de la délivrance du brevet: **07.03.2007**
(21) Numéro de dépôt: 01401079.7
(22) Date de dépôt: 26.04.2001
(51) Int. Cl.: C08J 3/03, C08J 3/07, A61K 47/32, A61Q 19/00, A61K 9/107

(54) **Latex inverses sur squalane ou polyisobutène hydrogéné et compositions cosmétiques, dermocosmétiques, dermopharmaceutiques ou pharmaceutiques en comportant**
Inverslatex basierend auf Squalan oder hydriertem Polyisobuten und diese enthaltende kosmetische Zusammensetzungen, Hautkosmetika, sowie pharmazeutische Zusammensetzungen
Inverse latex based on squalane or hydrogenated polyisobutene and cosmetic compositions, dermocosmetics, dermopharmaceuticals or pharmaceutical compositions containing them

(30) Priorité: 05.05.2000 FR 0005791
(43) Date de publication de la demande: 07.11.2001
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: Tabacchi, Guy, 81100 Castres (FR); Boiteux, Jean-Pierre, 81710 Saix (FR); Amalric, Chantal, 81700 Blan (FR); Michel, Nelly, 94700 Maisons Alfort (FR); Mallo, Paul, 78400 Chatou (FR)
(74) Mandataire: Conan, Philippe Claude

(56) Documents cités:
- EP-A- 1 010 708
- WO-A-00/32639
- FR-A- 2 721 511
- FR-A- 2 774 688
- FR-A- 2 774 996
- FR-A- 2 782 086
- FR-A- 2 785 801
- US-A- 5 185 395

## Description

La présente demande concerne des latex inverses eau dans huile, leur procédé de préparation et leur application en tant qu'épaississant et/ou émulsionnant pour des produits de soins de la peau et des cheveux ou pour la fabrication de préparations cosmétiques, dermocosmétiques, dermopharmaceutiques ou pharmaceutiques.

Des polymères épaississants synthétiques, se présentant sous forme de latex inverses, sont décrits comme pouvant être utilisés dans la fabrication de compositions topiques, dans les demandes de brevet français publiées sous les numéros 2721511, 2733805, 2774688, 2774996 et 2782086 ainsi que dans la demande de brevet européen publiée sous le numéro EP 0 503 853.

Parmi ceux-ci, la demande de brevet français .2 782 086 divulgue un latex inverse de coplymère de 2-méthyl-2[(1-oxo-2-propényl) amino] 1-propanesulfonate de sodium et d'acrylate de 2-hydroxy éthyle] réticulé au méthylène bis(acrylamide) dans l'isohexadécane, la demande de brevet français 2 774 688 divulgue un latex inverse de copolymère de 2-méthyl-2[(1-oxo-2-propényl) amino] 1-propanesulfonate de sodium et d'acrylate de sodium réticulé au méthylène bis(acrylamide) dans l'isohexadécane et la demande de brevet européen, qui appartient à l'état de la technique au sens de l'article 54(3) CBE, divulgue un latex inverse de polyacrylate d'ammonium ou de monoéthanolamine, réticulé au diallyoxyacétate de sodium ou au triallylamine dans l'isohexadécane.

Cependant, certains d'entre eux engendrent parfois des réactions d'intolérance de certaines peaux sensibles.

C'est pourquoi la demanderesse s'est intéressée à la recherche de nouvelles émulsions de polymères, qui soient mieux tolérées par la peau que celles de l'état de la technique.

L'invention a pour objet une composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids, et de préférence de 25% à 50% en poids, d'un polyélectrolyte branché ou réticulé, caractérisé en ce que ledit polyélectrolyte est un copolymère à base d'au moins un monomère possédant une fonction acide fort, copolymérisé soit avec au moins un monomère possédant une fonction acide faible, soit avec au moins un monomère neutre et caractérisé en ce que le solvant constitutif de la phase huile est choisi parmi le squalane ou le polyisobutène hydrogéné.

Le polyisobutène hydrogéné est commercialisé en France par la société Ets B. Rossow et Cie sous le nom PARLEAM - POLYSYNLANE™. Il est cité dans : Michel and Irene Ash ; Thesaurus of Chemical Products, Chemical Publishing Co , Inc. 1986, Volume I, page 211 (ISBN 0 7131 3603 0).

Le squalane est commercialisé en France par la société SOPHIM, sous le nom PHYTOSQUALAN™. Il est identifié dans Chemical Abstracts par le numéro RN = 111-01-3. C'est un mélange d'hydrocarbures contenant plus de 80% en poids de 2,6,10,15,19,23-hexaméthyl tétracosane.

Selon un deuxième aspect particulier de la présente invention, le solvant constitutif de la phase huile du latex inverse, est le polyisobutène hydrogéné.

Selon un troisième aspect particulier de la présente invention, le solvant constitutif de la phase huile du latex inverse, est le squalane.

Par polymère branché, on désigne un polymère non linéaire qui possède des chaînes pendantes de manière à obtenir, lorsque ce polymère est mis en solution dans l'eau, un fort état d'enchevêtrement conduisant à des viscosités à bas gradient très importantes.

Par polymère réticulé, on désigne un polymère non linéaire se présentant à l'état de réseau tridimensionnel insoluble dans l'eau, mais gonflable à l'eau et conduisant donc à l'obtention d'un gel chimique.

La composition selon l'invention peut comporter des motifs réticulés et/ou des motifs branchés.

Par "agent émulsifiant du type eau dans huile", on désigne des agents émulsifiants possédant une valeur de HLB suffisamment faible pour fournir des émulsions eau dans huile tels que les polymères tensioactifs commercialisés sous le nom de HYPERMER™ ou tels que les esters de sorbitan, comme le monooléate de sorbitan commercialisé par la Société SEPPIC sous le nom de marque MONTANE™ 80 ou l'isostéarate de sorbitan commercialisé par SEPPIC sous le nom de MONTANE™ 70. On peut aussi adjoindre à ces agents émulsifiants, l'oléate de sorbitan éthoxylé avec 5 moles d'oxyde d'éthylène, commercialisé par la société SEPPIC sous le nom de MONTANOX™ 81.

Par "agent émulsifiant du type huile dans eau", on désigne des agents émulsifiants possédant une valeur de HLB suffisamment élevée pour fournir des émulsions huile dans l'eau tels que les esters de sorbitan éthoxylés comme l'oléate de sorbitan éthoxylé avec 20 moles d'oxyde d'éthylène, commercialisé par la société SEPPIC sous le nom de MONTANOX™ 80, l'huile de ricin éthoxylée à 40 moles d'oxyde d'éthylène commercialisée par la société SEPPIC sous le nom de SIMULSOL™ OL50, le laurate de sorbitan éthoxylé à 20 moles d'oxyde d'éthylène commercialisé par la société SEPPIC sous le nom de MONTANOX™ 20 ou l'alcool laurique éthoxylé à 7 moles d'oxyde d'éthylène, commercialisé par la société SEPPIC sou le nom de SIMULSOL™ P7.

Comme agents émulsifiants possédant une valeur de HLB suffisamment élevée pour fournir des émulsions huile dans l'eau, il y a aussi les composés de formule (I) :

R₁-O-[CH(R₂)-CH₂-O]ₙ-(G)ₓ-H (I)

dans laquelle R₁ représente un radical hydrocarboné linéaire ou ramifié, saturé ou insaturé comprenant de 1 à 30 atomes de carbones, R₂ représente un atome d'hydrogène ou radical alkyle comprenant 1 ou 2 atomes de carbone, G représente le reste d'un saccharide, x représente un nombre décimal compris entre 1 et 5, et n est égal, soit à zéro, soit à un nombre entier compris entre 1 et 30.

Par reste d'un saccharide, on désigne pour G, un radical bivalent résultant de l'enlèvement sur une molécule de sucre, d'une part d'un atome d'hydrogène d'un groupe hydroxyle et d'autre part du groupe hydroxyle anomérique. Le terme saccharide désigne notamment le glucose ou dextrose, le fructose, le mannose, le galactose, l'altrose, l'idose, l'arabinose, le xylose, le ribose, le gulose, le lyxose, le maltose, le maltotriose, le lactose, le cellobiose, le dextrane, le talose, l'allose, le raffinose, le lévoglucane, la cellulose ou l'amidon. La structure oligomérique (G)ₓ peut se présenter sous toute forme d'isomérie, qu'il s'agisse d'isomérie optique, d'isomérie géométrique ou d'isomérie de position ; elle peut aussi représenter un mélange d'isomères.

Dans la formule (I) telle que définie précédemment, le radical R₁-O-[CH(R₂)-CH₂-O]ₙ- est lié à G, par le carbone anomérique de manière à former une fonction acétal. Le groupe divalent -[CH(R₂)-CH₂-O]ₙ- représente, soit une chaîne composée uniquement de groupes éthoxyle (R₂ = H), soit une chaîne composée uniquement de groupes propoxyle (R₂ = CH₃), soit une chaîne composée à la fois de groupes éthoxyle et de groupes propoxyle. Dans ce dernier cas, les fragments -CH₂-CH₂-O- et -CH(CH₃)-CH₂-O- sont distribués dans ladite chaîne, de façon séquencée ou aléatoire.

Le nombre x, qui représente dans la formule (I) le degré moyen de polymérisation du saccharide, est plus particulièrement compris entre 1 et 3, notamment entre 1,05 et 2,5, tout particulièrement entre 1,1 et 2,0 et de préférence, inférieur ou égal à 1,5.

Comme agents tensioactifs émulsifiants possédant une valeur de HLB suffisamment élevée pour fournir des émulsions huile dans l'eau, il y a plus particulièrement, les composés de formule (I) telle que définie précédemment, dans laquelle G représente le reste du glucose ou le reste du xylose et/ou dans laquelle n est égal à 0, et/ou dans laquelle R₁ représente un radical comportant de 8 à 18 atomes de carbone et plus particulièrement dans laquelle R₁ représente un radical choisis parmi les radicaux octyle, décyle, undécyle, dodécyle, tétradécyle ou hexadécyle, lesdits radicaux étant linéaires ou ramifiés.

Comme exemples de produits commerciaux contenant lesdits composés, il y a par exemple :
Le SIMULSOL™ SL8, commercialisé par la société SEPPIC, qui est une solution aqueuse contenant entre environ 35% et 45% en poids d'un mélange d'alkyl polyglycosides consistant en entre 45% en poids et 55% en poids d'un composé de formule (I), dans laquelle G représente le reste du glucose, x est égal à environ 1,45, n est égal à 0 et R₁ représente un radical décyle, et entre 45% en poids et 55% en poids d'un composé de formule (I), dans laquelle G représente le reste du glucose, x est égal à environ 1,45, n est égal à 0 et R₁ représente un radical octyle ;
Le SIMULSOL™ SL10, commercialisé par la société SEPPIC, qui est une solution aqueuse contenant entre environ 40% et poids et 50% en poids d'un mélange d'alkyl polyglycosides, consistant en environ 85% en poids d'un composé de formule (I), dans laquelle G représente le reste du glucose, x est égal à environ 1,45, n est égal à 0 et R₁ représente un radical décyle, environ 7,5% en poids d'un composé de formule (I), dans laquelle G représente le reste du glucose, x est égal à environ 1,45, n est égal à 0 et R₁ représente un radical dodécyle et environ 7,5% en poids d'un composé de formule (I), dans laquelle G représente le reste du glucose, x est égal à environ 1,45, n est égal à 0 et R₁ représente un radical tétradécyle ;
Le SIMULSOL™ SL11, commercialisé par la société SEPPIC, qui est une solution aqueuse contenant entre environ 40% et poids et 50% en poids d'un mélange d'alkyl polyglycosides de formule (I), dans laquelle G représente le reste du glucose, x est égal à environ 1,45, n est égal à 0 et R₁ représente un radical undécyle ; ou
Le SIMULSOL™ SL26, commercialisé par la société SEPPIC, qui est une solution aqueuse contenant entre environ 40% et poids et 55% en poids d'un mélange d'alkyl polyglycosides consistant en environ 70% en poids d'un composé de formule (I), dans laquelle G représente le reste du glucose, x est égal à environ 1,45, n est égal à 0 et R₁ représente un radical dodécyle, environ 25% en poids d'un composé de formule (I), dans laquelle G représente le reste du glucose, x est égal à environ 1,45, n est égal à 0 et R₁ représente un radical tétradécyle et environ 5% en poids d'un composé de formule (I), dans laquelle G représente le reste du glucose, x est égal à environ 1,45, n est égal à 0 et R₁ représente un radical hexadécyle.

La fonction acide fort du monomère en comportant est notamment la fonction acide sulfonique ou la fonction acide phosphonique, partiellement ou totalement salifiée. Ledit monomère peut être par exemple l'acide styrènesulfonique partiellement ou totalement salifié. Il est de préférence l'acide 2-méthyl 2-[(1-oxo 2-propènyl) amino] 1-propanesulfonique partiellement ou totalement salifiée sous forme d'un sel de métal alcalin tel que par exemple le sel de sodium ou le sel de potassium, de sel d'ammonium, d'un sel d'un amino alcool tel que par exemple le sel de monéthanolamine ou d'un sel d'amino acide tel que par exemple le sel de lysine.

La fonction acide faible du monomère en comportant est notamment la fonction acide carboxylique, et de préférence, ledit monomère est choisi parmi l'acide acrylique, l'acide méthacrylique, l'acide itaconique ou l'acide maléique partiellement ou totalement salifié.

Le monomère neutre est notamment choisi parmi l'acrylate de (2-hydroxy éthyle), l'acrylate de (2,3-dihydroxy propyle), le méthacrylate de (2-hydroxy éthyle), le méthacrylate de (2,3-dihydroxy propyle), ou un dérivé éthoxylé de poids moléculaire compris entre 400 et 1000, de chacun de ces esters.

Selon un quatrième aspect particulier de la présente invention, le polyélectrolyte compris dans le latex inverse tel que défini précédemment, est un copolymère comportant en proportions molaires entre 30% et 90% et plus particulièrement, entre 50% et 90% d'acide 2-méthyl 2-[(1-oxo 2-propènyl) amino] 1-propane-sulfonique partiellement ou totalement salifié, et entre 10% et 70% et plus particulièrement, entre 10% et 50% d'acrylate de (2-hydroxy éthyle). Le latex inverse tel que défini ci-dessus, comprend plus particulièrement de 20% en poids à 60% et de préférence de 25% à 45% en poids du copolymère tel défini ci- dessus. Il s'agit de préférence d'un latex inverse tel que défini ci-dessus, d'un copolymère comportant en proportions molaires de 60% à 90% de sel de sodium ou de sel d'ammonium de l'acide 2-méthyl 2-[(1-oxo 2-propènyl) amino] 1-propanesulfonique et de 1O% à 40% d'acrylate de (2-hydroxy éthyle).

Selon un cinquième aspect particulier de la présente invention, le polyélectrolyte compris dans le latex inverse tel que défini précédemment, est un copolymère comportant en proportions molaires entre 30% et 90% et plus particulièrement entre 30 et 45% de sel de sodium de sel d'ammonium, de sel de monoéthanolamine ou de sel de lysine de l'acide 2-méthyl 2-[(1-oxo 2-propènyl) amino] 1-propanesulfonique et de 10% à 70% et plus particulièrement de 55% à 70% d'acide acrylique partiellement ou totalement salifié sous forme de sel de sodium, de sel d'ammonium, de sel de monoéthanolamine ou de sel de lysine.

L'invention a plus particulièrement pour objet une composition telle que définie précédemment, caractérisée en ce que le polyélectrolyte est réticulé et/ou branché avec un composé diéthylénique ou polyéthylénique dans la proportion molaire exprimée par rapport aux monomères mis en oeuvre, de 0,005% à 1%, plus particulièrement de 0,01 % à 0,5 % et, tout particulièrement, de 0,1 % à 0,25 %.
L'agent de réticulation et/ou l'agent de ramification est choisi parmi l'acide diallyloxyacétique ou un de ses sels comme le diallyloxyacétate de sodium, le diméthacrylate d'éthylèneglycol, le diacrylate d'éthylèneglycol, le diallyl urée, le triméthylol propanetriacrylate, le méthylène-bis(acrylamide), le triallylamine ou un mélange de ces composés.

Le latex inverse tel que défini ci-dessus, contient généralement de 4% à 10% en poids, d'agents émulsifiants. Généralement de 20% à 50% et plus particulièrement de 25% à 40% du poids total des émulsifiants sont du type eau dans huile et de 80% à 50% et plus particulièrement de 75 à 60% sont du type huile dans eau.

Sa phase huile représente de 15% à 40%, et de préférence de 20% à 25%, de son poids total. Ce latex peut en outre contenir un ou plusieurs additifs choisis notamment parmi les agents complexants, les agents de transfert ou les agents limiteurs de chaînes.

L'invention a donc pour objet, une composition cosmétique, dermopharmaceutique ou pharmaceutique, caractérisée en ce qu'elle comprend au moins un composé épaississant, au moins un latex inverse tel que défini précédemment.

La composition cosmétique, dermocosmétique, dermopharmaceutique ou pharmaceutique définie ci-dessus comprend généralement de 0,1 % à 10 % et plus particulièrement entre 0,5% et 5% en poids dudit latex inverse. Elle se présente notamment, sous la forme d'un lait, d'une lotion, d'un gel, d'une crème, d'un savon, d'un bain moussant, d'un baume, d'un shampooing ou d'un après shampooing.

De façon générale, ledit latex inverse, peut remplacer avantageusement les produits vendus sous le nom SEPIGEL™ 305 ou SEPIGEL™ 501 par la demanderesse, dans les compositions cosmétiques, dermopharmaceutiques ou pharmaceutiques, car il présente aussi une bonne compatibilité avec les autres excipients utilisés pour la préparation de formulations telles que les laits, les lotions, les crèmes, les savons, les bains, les baumes, les shampooings ou les après shampooings. Il peut aussi être utilisé en combinaison lesdits SEPIGEL Il est notamment compatible avec les concentrés décrits et revendiqués dans les publications internationales WO 92/06778, WO 95/04592, W095/13863, WO 98/47610 ou FR 2734 496 ou avec les agents tensioactifs décrits dans WO 93/08204. Il est particulièrement compatible avec le MONTANOV™ 68, le MONTANOV™ 82, le MONTANOV™ 202 le MONTANOV™ WO18, le MONTANOV™ S ou le SEPIPERL™ N. Il peut également être utilisé dans des émulsions du type de celles décrites et revendiquées dans EP 0 629 396 et dans les dispersions aqueuses cosmétiquement ou physiologiquement acceptables avec un composé organo-polysiloxane choisi, par exemple parmi ceux décrits dans WO 93/05762 ou dans WO 93/21316. Il peut également être utilisé pour former des gels aqueux à pH acide cosmétiquement ou physiologiquement acceptables, tels que ceux décrit dans WO 93/07856 ; il peut encore être utilisé en association avec des celluloses non-ioniques, pour former par exemple des gels de coiffage, tels que ceux décrits dans EP 0 684 024 ou encore en association avec des esters d'acides gras et de sucre, pour former des compositions pour le traitement du cheveux ou de la peau telles que celles décrites dans EP 0 603 019. ou encore dans les shampooings ou après shampooings tels que décrits et revendiqués dans WO 92/21316 ou enfin en association avec un homo polymère anionique tels que le CARBOPOL™ pour former des produits de traitement des cheveux comme ceux décrits dans DE 195 23596. Il est également compatible avec de nombreux principes actifs, tels que par exemple, les agents autobronzants comme le dihydroxyacétone (DHA) ou les agents anti-acné ; il peut donc être introduit dans des compositions auto-bronzantes comme celles revendiquées dans EP 0 715 845, EP 0604249,
EP 0576188 ou dans WO 93/07902. Il est également compatible avec les dérivés N-acylés d'aminoacides, ce qui permet son utilisation dans des compositions apaisantes notamment pour peaux sensibles, telles que celles décrites ou revendiquées dans WO 92/21318, WO 94/27561 ou WO 98/09611. Il est aussi compatible avec les acides glycoliques, avec l'acide lactique, avec l'acide salicylique les rétinoïdes, le phénoxy éthanol, les sucres, le glycéraldéhyde, les xanthanes, les acides de fruit, et les divers polyols utilisés dans la fabrication de formulations cosmétiques.

L'invention a donc aussi pour objet, l'utilisation d'un latex inverse tel que défini précédemment, pour préparer une composition cosmétique, dermocosmétique, dermopharmaceutique ou pharmaceutique.

Les exemples qui suivent ont pour but d'illustrer la présente invention sans toutefois la limiter. Ils montrent que les nouveaux latex inverses n'irritent pas la peau et que leurs propriétés physiques permettent leur utilisation dans la préparation de compositions cosmétiques, dermopharmaceutiques ou pharmaceutiques plus particulièrement destinées au traitement des peaux sensibles.

### A) Exemples de préparations de compositions selon l'invention

### Exemple 1 : Latex inverse d'un copolymère AMPS (sel de Na) / acide acrylique (sel de Na), réticulé au méthylène bis(acrylamide), dans le polyisobutène hydrogéné (Composition 1)

**a**) - On charge dans un bécher sous agitation :
   - 61,4 g d'acide acrylique,
   - 470,2 g d'une solution commerciale à 55% de 2-méthyl 2-[(1-oxo 2-propènyl) amino] 1-propanesulfonate de sodium,
   - 35,54 g d'une solution aqueuse à 48% en poids d'hydroxyde de sodium,
   - 0,45 g d'une solution aqueuse à 40% en poids de diéthylènetriaminepentacétate de sodium et
   - 0,128 g de méthylène bis(acrylamide).

   Le pH de cette solution aqueuse est ajustée à 5,1 et l'on ajoute de l'eau permutée de manière à porter la masse de la phase aqueuse à 643,8 g.
**b**) - On prépare une phase organique en mélangeant :
   - 260 g de polyisobutène hydrogéné,
   - 30,7 g de MONTANE™ 80 VG,
   - 0,2 g d'azo bis(isobutyronitrile).
**c**) - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 5 ml d'une solution contenant 0,75% en poids d'hydroperoxyde de cumène dans le polyisobutène hydrogéné, puis, après homogénéisation de la solution, une solution aqueuse de métabisulfite de sodium 2,5 g dans 100 ml d'eau) à raison de 0,5 ml / minute pendant environ 60 minutes et en laissant monter la température jusqu'à la température de polymérisation. On maintient alors le milieu réactionnel pendant environ 90 minutes à cette température, à l'issu desquelles le mélange obtenu est refroidi jusqu'à 35° environ. On introduit lentement 50 g d'oléate de sorbitan éthoxylé à 20 moles (MONTANOX™ 80) et on obtient l'émulsion eau dans huile désirée.

### Propriétés physiques

Viscosité dans l'eau à 3% du latex (Brookfield RVT Mobile 6, vitesse 5) :
η = 89 200 mPas
Viscosité à 3% de latex + dans de l'eau salée (NaCl 0,1%) (Brookfield RVT Mobile 3, vitesse 5) : η = 10 500 mPas.

### Exemple 2 : Latex inverse d'un copolymère AMPS (sel de Na) / acide acrylique (sel de Na), réticulé au méthylène bis(acrylamide), dans le polyisobutène hydrogéné (Composition 2).

**a**) - On charge dans un bécher sous agitation :
   - 61,4 g d'acide acrylique,
   - 470,2 g d'une solution commerciale à 55% de 2-méthyl 2-[(1-oxo 2-propènyl) amino] 1-propanesulfonate de sodium,
   - 35,54 g d'une solution aqueuse à 48% en poids d'hydroxyde de sodium,
   - 0,45 g d'une solution aqueuse à 40% en poids, de diéthylènetriaminepentacétate de sodium et
   - 0,128 g de méthylène bis(acrylamide).

   Le pH de cette solution aqueuse est ajustée à 5,1 et on rajoute de l'eau permutée de manière à porter la masse de la phase aqueuse à 643,8 g.
**b**) - On prépare une phase organique en mélangeant :
   - 260 g de polyisobutène hydrogéné,
   - 30,7 g de MONTANE™ 80 VG,
   - 0,2 g d'azo bis(isobutyronitrile).
**c**) - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 5 ml d'une solution contenant 0,75% en poids d'hydroperoxyde de cumène dans le polyisobutène hydrogéné, puis, après homogénéisation de la solution, une solution aqueuse de métabisulfite de sodium (2,5 g dans 100 ml d'eau) à raison de 0,5 ml /minute pendant environ 60 minutes et en laissant monter la température jusqu'à la température de polymérisation. On maintient alors le milieu réactionnel pendant environ 90 minutes à cette température, à l'issu desquelles le mélange obtenu est refroidi jusqu'à 35° environ. On introduit lentement 54,5 g de SIMULSOL™ SL8 et on obtient l'émulsion eau dans huile désirée.

### Propriétés physiques

Viscosité dans l'eau à 3% du latex (Brookfield RVT Mobile 6, vitesse 5) :
η = 87 600 mPas
Viscosité à 3% de latex + dans de l'eau salée (NaCI 0,1%) (Brookfield RVT Mobile 3, vitesse 5) : η = 16 800 mPas.

### Exemple 3 : Latex inverse d'un AMPS (sel de Na) /acide acrylique (sel de Na), réticulé au méthylène bis(acrylamide), dans le polyisobutène hydrogéné (Composition 3).

**a**) - On charge dans un bécher sous agitation :
   - 61,4 g d'acide acrylique,
   - 470,2 g d'une solution commerciale à 55% de 2-méthyl 2-[(1-oxo 2-propènyl) amino] 1-propanesulfonate de sodium,
   - 35,54 g d'une solution aqueuse à 48% en poids d'hydroxyde de sodium,
   - 0,45 g d'une solution aqueuse à 40% en poids de diéthylènetriaminepentacétate de sodium et
   - 0,128 g de méthylène bis(acrylamide).

   Le pH de cette solution aqueuse est ajustée à 5,1 et on rajoute de l'eau permutée de manière à porter la masse de la phase aqueuse à 643,8 g.
**b**) - On prépare une phase organique en mélangeant :
   - 260 g de polyisobutène hydrogéné,
   - 30,7 g de MONTANE™ 80 VG,
   - 0,2 g d'azo bis(isobutyronitrile).
**c**) - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 5 ml d'une solution contenant 0,75% en poids d'hydroperoxyde de cumène dans le polyisobutène hydrogéné, puis, après homogénéisation de la solution, une solution aqueuse de métabisulfite de sodium (2,5 g dans 100 ml d'eau) à raison de 0,5 ml /minute pendant environ 60 minutes et en laissant monter la température jusqu'à la température de polymérisation. On maintient alors le milieu réactionnel pendant environ 90 minutes à cette température, à l'issu desquelles le mélange obtenu est refroidi jusqu'à 35° environ. On introduit lentement 72,7 g de SIMULSOL™ SL26 et on obtient l'émulsion eau dans huile désirée.

### Propriétés physiques

Viscosité dans l'eau à 3% du latex (Brookfield RVT Mobile 6, vitesse 5) :
η = 79 600 mPas
Viscosité à 3% de latex + dans de l'eau salée (NaCI 0,1%) (Brookfield RVT Mobile 3, vitesse 5) : η = 10 600 mPas.

### Exemple 4 : Latex inverse d'un copolymère AMPS (sel de lysine) /acide acrylique (sel de lysine), réticulé au méthylène bis(acrylamide), dans le squalane (Composition 4).

**a**) - On charge dans un bécher sous agitation :
   - 61,4 g d'acide acrylique,
   - 470,2 g d'une solution commerciale à 55% de 2-méthyl 2-[(1-oxo 2-propènyl) amino] 1-propanesulfonate de sodium,
   - 0,46 g d'une solution aqueuse à 40% en poids de diéthylènetriaminepentacétate de sodium,
   - 0,161 g de méthylène bis(acrylamide),
   - 70, 0 g de L-Lysine et
   - 76,13 g d'eau.

   La quantité de L-Lysine est ajustée pour obtenir une valeur du pH de la solution proche de 5,0. La vitesse d'addition est telle que la température du milieu réactionnel n'excède pas 25°C.
**b**) - On prépare une phase organique en mélangeant :
   - 234,5 g de squalane,
   - 41,4 g de MONTANE™ 80 VG,
   - 0,2 g d'azo bis(isobutyronitrile).
**c**) - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 10 ml d'une solution contenant 0,28% en poids d'hydroperoxyde de cumène dans le squalane puis, après homogénéisation de la solution, une solution aqueuse de métabisulfite de sodium (2,5 g dans 100 ml d'eau) à raison de 0,5 ml / minute pendant environ 60 minutes et en laissant monter la température jusqu'à 75°C. On maintient alors le milieu réactionnel pendant environ 60 minutes à cette température, à l'issu desquelles le mélange obtenu est refroidi jusqu'à 35° environ. On introduit lentement 50 g de MONTANOX™ 80 et on obtient l'émulsion eau dans huile désirée.

### Propriétés physiques

Viscosité dans l'eau à 3% du latex (Brookfield RVT Mobile 6, vitesse 5) :
η = 51 000 mPas
Viscosité à 3% de latex dans un solution aqueuse de sel (0,1% NaCl) (Brookfield RVT Mobile 3, vitesse 5) : η = 6 700 mPas
pH de la solution aqueuse à 3% de latex : 6,3

### Exemple 5 : Latex inverse d'un copolymère AMPS (sel de monoéthanolamine) /acide acrylique (sel de monoéthanolamine), réticulé au méthylène bis(acrylamide), dans le squalane (Composition 5).

**a**) - On charge dans un bécher sous agitation :
   - 61,4 g d'acide acrylique,
   - 470,2 g d'une solution commerciale à 55% de 2-méthyl 2-[(1-oxo 2-propènyl) amino] 1-propanesulfonate de sodium,
   - 0,46 g d'une solution aqueuse à 40% en poids de diéthylènetriaminepentacétate de sodium et
   - 0,161 g de méthylène bis(acrylamide),
   - 26,0 g de monoéthanolamine et
   - 90,4 g d'eau.
**b**) - On prépare une phase organique en mélangeant :
   - 234,5 g de squalane,
   - 41,4 g de MONTANE™ 80 VG,
   - 0,2 g d'azo bis(isobutyronitrile).
**c**) - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 10 ml d'une solution contenant 0,28% en poids d'hydroperoxyde de cumène dans le squalane puis, après homogénéisation de la solution, une solution aqueuse de métabisulfite de sodium (2,5 g dans 100 ml d'eau) à raison de 0,5 ml / minute pendant environ 60 minutes et en laissant monter la température jusqu'à 75°C. On maintient alors le milieu réactionnel pendant environ 60 minutes à cette température, à l'issu desquelles le mélange obtenu est refroidi jusqu'à 35° environ. On introduit lentement 50 g de MONTANOX™ 80 et on obtient l'émulsion eau dans huile désirée.

### Propriétés physiques

Viscosité dans l'eau à 3% du latex (Brookfield RVT Mobile 6, vitesse 5) :
η = 69 200 mPas
Viscosité à 3% de latex dans un solution aqueuse de sel (0,1% NaCI) (Brookfield RVT Mobile 3, vitesse 5) : η = 6 300 mPas
pH de la solution aqueuse à 3% de latex : 6,0

### Exemple 6 : Latex inverse d'un copolymère AMPS (sel de Na) / acrylate de (2-hydroxy éthyle), réticulé au méthylène bis(acrylamide), dans le squalane (Composition 6).

**a**) - On charge dans un bécher sous agitation :
   - 20,4 g d'acrylate de (2-hydroxy éthyle),
   - 660,6 g d'une solution commerciale à 55% de 2-méthyl 2-[(1-oxo 2-propènyl) amino] 1-propanesulfonate de sodium,
   - 0,45 g d'une solution aqueuse à 40% en poids, de diéthylènetriaminepentacétate de sodium et
   - 0,123 g de méthylène bis(acrylamide).

   On ajuste le pH à 4,0 en ajoutant 0,55 g d'acide 2-méthyl 2-[(1-oxo 2-propènyl) amino] 1-propanesulfonique.
**b**) - On prépare une phase organique en mélangeant :
   - 265, g de squalane,
   - 17,76 g de MONTANE™ 80 VG,
   - 9,24 g de MONTANOX™ 81 VG
   - 0,2 g d'azo bis(isobutyronitrile).
**c**) - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 10 ml d'une solution contenant 0,28 % en poids d'hydroperoxyde de cumène dans le squalane puis, après homogénéisation de la solution, une solution aqueuse de métabisulfite de sodium (2,5 g dans 100 ml d'eau) à raison de 0,5 ml / minute pendant environ 60 minutes et en laissant monter la température jusqu'à 75°C. On maintient alors le milieu réactionnel pendant environ 60 minutes à cette température, à l'issu desquelles le mélange obtenu est refroidi jusqu'à 35° environ. On introduit lentement 25 g de MONTANOX™ 80 et on obtient l'émulsion eau dans huile désirée.

### Propriétés physiques

Viscosité dans l'eau à 3% du latex (Brookfield RVT Mobile 6, vitesse 5) :
η = 90 000 mPas
Viscosité à 3% de latex dans une solution aqueuse de sel (0,1% NaCl)à pH = 3 (Brookfield RVT Mobile 6, vitesse 5) : η = 58 800 mPas
pH de la solution aqueuse à 3% de latex : 5,3

### Exemple 7: Latex inverse d'un copolymère AMPS (sel de Na) / acrylate de (2-hydroxy éthyle), réticulé au méthylène bis(acrylamide), dans le squalane (Composition 7).

**a**) - On charge dans un bécher sous agitation :
   - 20,4 g d'acrylate de (2-hydroxy éthyle),
   - 660,6 g d'une solution commerciale à 55% de 2-méthyl 2-[(1-oxo 2-propènyl) amino] 1-propanesulfonate de sodium,
   - 0,45 g d'une solution aqueuse à 40% en poids de diéthylènetriaminepentacétate de sodium et
   - 0,123 g de méthylène bis(acrylamide).

   On ajuste le pH à 4,0 en ajoutant 0,55 g d'acide 2-méthyl 2-[(1-oxo 2-propènyl) amino] 1-propanesulfonique.
**b**) - On prépare une phase organique en mélangeant :
   - 265, g de squalane,
   - 17,76 g de MONTANE™ 80 VG,
   - 9,24 g de MONTANOX™ 81 VG
   - 0,2 g d'azo bis(isobutyronitrile).
**c**) - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 10 ml d'une solution contenant 0,28 % en poids d'hydroperoxyde de cumène dans le squalane puis, après homogénéisation de la solution, une solution aqueuse de métabisulfite de sodium (2,5 g dans 100 ml d'eau) à raison de 0,5 ml /minute pendant environ 60 minutes et en laissant monter la température jusqu'à 75°C. On maintient alors le milieu réactionnel pendant environ 60 minutes à cette température, à l'issu desquelles le mélange obtenu est refroidi jusqu'à 35° environ. On introduit lentement 27,2 g de SIMULSOL™ SL10 et on obtient l'émulsion eau dans huile désirée.

### Propriétés physiques

Viscosité dans l'eau à 3% du latex (Brookfield RVT Mobile 6, vitesse 5) :
η = 113 000 mPas
Viscosité à 3% de latex dans une solution aqueuse de sel (0,1% NaCl) (Brookfield RVT Mobile 3, vitesse 5) : η = 17 200 mPas
pH de la solution aqueuse à 3% de latex : 5,8

### Exemple 8 : Latex inverse d'un copolymère AMPS (sel de Na) / acrylate de (2-hydroxy éthyle), réticulé au méthylène bis(acrylamide), dans le squalane (Composition 8).

**a**) - On charge dans un bécher sous agitation :
   - 20,4 g d'acrylate de (2-hydroxy éthyle),
   - 660,6 g d'une solution commerciale à 55% de 2-méthyl 2-[(1-oxo 2-propènyl) amino] 1-propanesulfonate de sodium,
   - 0,45 g d'une solution aqueuse à 40% en poids, de diéthylènetriaminepentacétate de sodium et
   - 0,123 g de méthylène bis(acrylamide).

   On ajuste le pH à 4,0 en ajoutant 0,55 g d'acide 2-méthyl 2-[(1-oxo 2-propènyl) amino] 1-propanesulfonique.
**b**) - On prépare une phase organique en mélangeant :
   - 265, g de squalane,
   - 17,76 g de MONTANE™ 80 VG,
   - 9,24 g de MONTANOX™ 81 VG
   - 0,2 g d'azo bis(isobutyronitrile).
**c**) - La phase aqueuse est introduite progressivement dans la phase organique et l'ensemble est agité fortement au moyen d'un agitateur ULTRA-TURRAX™ commercialisé par IKA. L'émulsion obtenue est alors transférée dans un réacteur de polymérisation, soumise à un barbotage d'azote puis refroidit à environ 5-6°C. On ajoute alors 10 ml d'une solution contenant 0,28% en poids, d'hydroperoxyde de cumène dans le squalane puis, après homogénéisation de la solution, une solution aqueuse de métabisulfite de sodium (2,5 g dans 100 ml d'eau) à raison de 0,5 ml / minute pendant environ 60 minutes et en laissant monter la température jusqu'à 75°C. On maintient alors le milieu réactionnel pendant environ 60 minutes à cette température, à l'issu desquelles le mélange obtenu est refroidi jusqu'à 35° environ. On introduit lentement 27,2 g de SIMULSOL™ SL8 et on obtient l'émulsion eau dans huile désirée.

### Propriétés physiques

Viscosité dans l'eau à 3% du latex (Brookfield RVT Mobile 6, vitesse 5) :
η = 93 000 mPas
Viscosité à 3% de latex dans une solution aqueuse de sel (0,1% NaCl) (Brookfield RVT Mobile 6, vitesse 5) : η = 6 100 mPas
pH de la solution aqueuse à 3% de latex : 5,8

### B Propriétés des compositions selon l'invention

### a) Stabilité à la température

On a préparé un gel crème comprenant 3% de composition 6 et 20% de cétéaryl octanoate et mesuré la viscosité Les résultats sont les suivants:

| | Viscosité Brookfield LVT 6rpm (en mPas) | |
|---|---|---|
| | A température ambiante | A 50°C |
| Après 1 jour | 90 000 mPas | 90 000 mPas |
| Après 7 jours | 90 000 mPas | 90 000 mPas |
| Après 1 mois | 90 000 mPas | 90 000 mPas |

### b) Stabilité au rayonnement UV

On constate que le gel préparé avec la composition 6 est très stable UV; car sa viscosité n'a pas varié après 14 jours d'exposition.

### c) Influence du pH sur la viscosité

La viscosité du gel crème préparé avec la composition 6 est très stable au pH dans l'intervalle pH = 3 à pH = 8

### d) Etude comparative de la tolérance cutanée

La tolérance locale épicutanée d'une série de gels-crèmes, contenant 3 % et 5 % en poids d'une des compositions 1 à 8 préparées comme décrit ci-dessus, a été déterminée et comparée à celle observée avec un latex inverse d'un copolymère AMPS/acrylate de réticulé au méthylène bis(acrylamide), dans l'isohexadécane (Composition A). selon le protocole suivant :

On applique la composition à tester, une surface d'environ 50 mm² de la région sous-capulaire gauche, de la peau du dos de 38 volontaires sains, parmi lesquels 19 ont une peau de type "peau japonaise" (PJ) et 19 une peau de type "peau caucasienne" (PC). Le contact est maintenu pendant 48 heures sous timbre occlusif.

Cette application est aussi réalisée dans les mêmes conditions avec un timbre seul (sans composition) en tant que témoin négatif.

L'observation clinique de la surface de peau ainsi traitée est réalisée 30 minutes puis 24 heures après la dépose desdits timbres. Ces observations sont faites par comparaison à la surface non traitée témoin négatif.

La quantification de l'irritation cutanée, selon une échelle numérique allant de 0 à 4 (0 : pas d'effet ; 1 : effet très léger ; 2 : effet net ; 3 et 4: effet modéré à sévère selon les réactions), est réalisée pour chacune des réactions éventuellement observées à savoir : érythème, oedème, vésicules, sécheresse de la peau, rugosité de la peau et la réflectivité de la peau.

Les indices de tolérance cutanée (IC), expriment la moyenne de la somme des effets quantifiés relevés sur chaque volontaire :
IC = 0 signifie qu'aucune irritation n'a été observée,
IC ≤ 0,5 signifie que le produit est statistiquement bien toléré,
IC > 0,5 signifie que le produit engendre une intolérance.
Les résultats exprimés, en indices de tolérance cutanée, sont consignés dans le tableau suivant :

| | Indice de tolérance cutanée | | | |
|---|---|---|---|---|
| | gel à 3% | | gel à 5% | |
| | P.J. | P.C. | P.J. | P.C. |
| Composition 1 | n.e. | 0,00 | n.e. | 0,00 |
| Composition 2 | 0,89 | 0,21 | 0,63 | 0,05 |
| Composition 3 | 0,89 | 0,21 | 0,63 | 0,05 |
| Composition 4 | 1,00 | 0,05 | 1,11 | 0,05 |
| Composition 5 | n.e. | n.e. | n.e. | n.e. |
| Composition 6 | 1,00 | 0,00 | 1,11 | 0,05 |
| Composition 7 | n.e. | 0,1 | n.e. | 0,00 |
| Composition 8 | 0,53 | 0,0 | 0,95 | 0,00 |
| Composition A | 1,00 | 0,47 | 0,95 | 0,47 |

Ces résultats montrent que de façon inattendue, le squalane et, le polyisobutène hydrogéné potentialisent la tolérance cutanée polymère du latex inverse.

### C) Exemples de formulations préparées avec les compositions selon l'invention

### Exemple 9 : Crème de soin

| | |
|---|---|
| Cyclométhicone : | 10% |
| Composition 3 : | 0,8% |
| MONTANOV™ 68 : | 2% |
| alcool stéarylique : | 1% |
| alcool stéarique : | 0,5% |
| conservateur : | 0,65% |
| Lysine: | 0,025% |
| EDTA (sel disodique) : | 0,05% |
| Gomme de xanthane : | 0,2% |
| Glycérine : | 3 % |
| Eau : | q.s.p. 100% |

### Exemple 10 : Baume après-rasage

### FORMULE

| | | |
|---|---|---|
| A | Composition 3 : | 1,5% |
| | Eau : | q.s.p. 100% |
| B | MICROPEARL™ M 100 : | 5,0% |
| | SEPICIDE™ CI : | 0,50% |
| | Parfum : | 0,20% |
| | éthanol 95° : | 10,0% |

### MODE OPERATOIRE

Ajouter B dans A.

### Exemple 11 : Emulsion satinée pour le corps

### FORMULE

| | | |
|---|---|---|
| A | SIMULSOL™ 165 : | 5,0% |
| | LANOL™ 1688 : | 8,50% |
| | beurre de Karité : | 2% |
| | huile de paraffine : | 6,5% |
| | LANOL™ 14M : | 3% |
| | LANOL™ S : | 0,6% |
| B | eau : | 66,2% |
| C | MICROPEARL™ 100 : | 5% |
| D | Composition 2 : | 3% |
| E | SEPICIDE™ CI : | 0,3% |
| | SEPICIDE™ HB : | 0,5% |
| | MONTEINE™ CA : | 1% |
| | Parfum : | 0,20% |
| | acétate de vitamine E : | 0,20% |
| | Sodium pyrolidinonecarboxylate : | 1% |

### MODE OPERATOIRE

Ajouter C dans B, émulsionner B dans A à 70°C, puis ajouter D à 60°C puis E à 30°C.

### Exemple 12 : Crème H/E

### FORMULE

| | | |
|---|---|---|
| A | SIMULSOL™ 165 : | 5,0% |
| | LANOL™ 1688: | 20,0% |
| | LANOL™ P : | 1,0% |
| B | eau : | q.s.p. 100% |
| C | Composition 2 : | 2,50% |
| D | SEPICIDE™ CI : | 0,20% |
| | SEPICIDE™ HB : | 0,30% |

### MODE OPERATOIRE

Introduire B dans A vers 75°C ; ajouter C vers 60°C, puis D vers 45°C.

### Exemple 13 : gel solaire non gras

### FORMULE

| | | |
|---|---|---|
| A | Composition 4 : | 3,00% |
| | Eau : | 30% |
| B | SEPICIDE™ Cl : | 0,20% |
| | SEPICIDE™ HB : | 0,30% |
| | Parfum : | 0,10% |
| C | colorant : | q.s.p. |
| | eau: | 30% |
| D | MICROPEARL™ M 100 : | 3,00% |
| | Eau : | q.s.p. 100% |
| E | huile de silicone : | 2,0% |
| | PARSOL™ MCX : | 5,00% |

### MODE OPERATOIRE

Introduire B dans A; ajouter C, puis D, puis E.

### Exemple 14 : Gel de massage

### FORMULE

| | | |
|---|---|---|
| A | Composition 1 : | 3,5% |
| | Eau : | 20,0% |
| B | colorant : | 2 gouttes/100g |
| | Eau : | q. s. |
| C | alcool: | 10% |
| | Menthol : | 0,10% |
| D | huile de silicone : | 5,0% |

### MODE OPERATOIRE

Ajouter B dans A, puis ajouter au mélange, C puis D

### Exemple 15 : Fond de teint hydratant et matifiant

### FORMULE

| | | |
|---|---|---|
| A | eau : | 20,0% |
| | Butylène glycol : | 4,0% |
| | PEG-400 : | 4,0% |
| | PECOSIL™ PS100 : | 1,0% |
| | NaOH : | q.s. pH = 9 |
| | Dioxyde de titane : | 7,0% |
| | Talc : | 2,0% |
| | Oxyde de fer jaune : | 0,8% |
| | Oxyde de fer rouge : | 0,3% |
| | Oxyde de fer noir: | 0,05% |
| B | LANOL™ 99 : | 8% |
| | Caprylic capric triglycéride | 8% |
| | MONTANOV™ 202 : | 5,00 % |
| C | eau : | q.s.p. 100% |
| | MICROPEARL™ M305 : | 2,0% |
| | EDTA tétrasodé : | 0,05% |
| D | Cyclométhicone : | 4,0% |
| | Gomme de Xanthane : | 0,2% |
| | composition 6 : | 0,8% |
| E | SEPICIDE™ HB : | 0,5% |
| | SEPICIDE Cl : | 03% |
| | Parfum : | 0,2% |

### MODE OPERATOIRE

préparer à 80°C, les mélanges B + D et A + C, puis mélanger et émulsionner l'ensemble.

### Exemple 16 : Gel coup d'éclat

### FORMULE

| | | |
|---|---|---|
| A | Composition 5 : | 4% |
| | Eau : | 30% |
| B | ELASTINE HPM : | 5,0% |
| C | MICROPEARL™ M 100 : | 3% |
| | Eau : | 5% |
| D | SEPICIDE™ Cl : | 0,2% |
| | SEPICIDE™ HB : | 0,3% |
| | Parfum : | 0,06% |
| Sodium pyrolidinonecarboxylate 50% : | | 1% |
| Eau : | | q. s. p. 100% |

### MODE OPERATOIRE

Préparer A ; additionner B, puis C, puis D.

### Exemple 17 : Lait corporel

### FORMULE

| | |
|---|---|
| MONTANOV™ S : | 3,5% |
| LANOL™ 37T : | 8,0% |
| SOLAGUM™ L : | 0,05% |
| Eau : | q.s.p.100% |
| Benzophénone: | 2,0% |
| diméthicone 350cPs : | 0,05% |
| Composition 4 : | 0,8% |
| conservateur: | 0,2% |
| parfum: | 0,4% |

### Exemple 18 : Emulsion démaquillante à l'huile d'amandes douces

### FORMULE

| | |
|---|---|
| MONTANOV™ 68 : | 5% |
| huile d'amandes douces : | 5% |
| eau : | q.s.p.100% |
| Composition 8 : | 0,3% |
| glycérine : | 5% |
| conservateur : | 0,2% |
| parfum : | 0,3% |

### Exemple 19 : Crème hydratante pour peaux grasses

### FORMULE

| | |
|---|---|
| MONTANOV™ 68 : | 5% |
| Cétylstéaryloctanoate : | 8% |
| octyl palmitate : | 2% |
| eau : | q.s.p.100% |
| Composition 4 : | 0,6% |
| MICROPEARL™ M100 : | 3,0% |
| Mucopolysaccharides : | 5% |
| SEPICIDE™ HB : | 0,8% |
| Parfum : | 0,3% |

### Exemple 20 : Baume après-rasage apaisant sans alcool

### FORMULE

| | | |
|---|---|---|
| A | LIPACIDE™ PVB : | 1,0% |
| | LANOL™ 99 : | 2,0% |
| | Huile d'amandes douces : | 0,5% |
| B | Composition 7 : | 3,5% |
| C | eau : | q.s.p.100% |
| D | parfum : | 0,4% |
| | SEPICIDE™ HB : | 0,4% |
| | SEPICIDE™ Cl : | 0,2% |

### Exemple 21 : Crème aux AHA pour peaux sensibles

### FORMULE

| | |
|---|---|
| mélange de lauryl aminoacides : | 0,1% à 5% |
| aspartate de magnésium et de potassium : | 0,002% à 0,5% |
| LANOL™ 99 : | 2% |
| MONTANOV™ 68 : | 5,0% |
| Eau : | q.s.p.100% |
| Composition 6 : | 1,50% |
| acide gluconique : | 1,50% |
| tri éthylamine : | 0,9% |
| SEPICIDE™ HB : | 0,3% |
| SEPICIDE™ Cl : | 0,2% |
| Parfum : | 0,4% |

### Exemple 22 : Lait démaquillant

### FORMULE

| | |
|---|---|
| SEPIPERL™ N : | 3% |
| PRIMOL™ 352 : | 8,0% |
| huile d'amandes douces : | 2 % |
| eau : | q.s.p.100% |
| Composition 6 : | 0,8% |
| conservateur: | 0,2% |

### Exemple 23 : Emulsion fluide à pH alcalin

| | |
|---|---|
| MARCOL™ 82 : | 5,0% |
| NaOH : | 10,0% |
| Eau : | q.s.p.100% |
| Composition 7 : | 1,5% |

### Exemple 24 : Fond de teint fluide

### FORMULE

| | |
|---|---|
| SIMULSOL™ 165: | 5,0% |
| LANOL™ 84D : | 8,0% |
| LANOL™ 99 : | 5,0% |
| Eau : | q.s.p.100% |
| pigments et charges minérales : | 10,0% |
| Composition 1 : | 1,2% |
| conservateur : | 0,2% |
| parfum : | 0,4% |

### Exemple 25 : Lait solaire

### FORMULE

| | |
|---|---|
| SEPIPERL™ N : | 3,5% |
| LANOL™ 37T : | 10,0% |
| PARSOL™ MCX : | 5,0% |
| EUSOLEX™ 4360 : | 2,0% |
| Eau : | q.s.p.100% |
| Composition 5 : | 1,8% |
| conservateur : | 0,2% |
| parfum : | 0,4% |

### Exemple 26 : Gel contour des yeux

### FORMULE

| | |
|---|---|
| Composition 6 : | 2,0% |
| Parfum : | 0,06% |
| Sodium pyrrolidinonecarboxylate : | 0,2% |
| DOW CORNING™ 245 Fluid : | 2,0% |
| Eau : | q. s. p. 100% |

### Exemple 27 : Composition de soin non rincée

### FORMULE

| | |
|---|---|
| Composition 4 : | 1,5% |
| Parfum : | q. s |
| Conservateur: | q. s. |
| DOW CORNING™ X2 8360 : | 5,0% |
| DOW CORNING™ Q2 1401 : | 15,0% |
| Eau : | q.s.p. 100% |

### Exemple 28 : Gel amincissant

| | |
|---|---|
| Composition 6 : | 5 % |
| Ethanol : | 30 % |
| Menthol : | 0,1 % |
| Caféine : | 2,5 % |
| extrait de ruscus : | 2 % |
| extrait de lierre : | 2 % |
| SEPICIDE™ HP : | 1 % |
| Eau: | q. s. p. 100 % |

### Exemple 29 : Gel crème teinté ultra naturel

### FORMULE

| | | |
|---|---|---|
| A | eau : | 10,0% |
| | Butylène glycol : | 4,0% |
| | PEG-400 : | 4,0% |
| | PECOSIL™ PS100 : | 1,5% |
| | NaOH : | q.s. pH = 7 |
| | Dioxyde de titane : | 2,0% |
| | Oxyde de fer jaune : | 0,8% |
| | Oxyde de fer rouge : | 0,3% |
| | Oxyde de fer noir: | 0,05% |
| B | LANOL™ 99 : | 4,0% |
| | Caprylic capric triglycéride | 4,0% |
| | SEPIFEEL™ ONE : | 1,0% |
| | composition 8 : | 3,0% |
| C | eau : | q.s.p. 100% |
| | MICROPEARL™ M305 : | 2,0% |
| | EDTA tétrasodé : | 0,05% |
| | Cyclométhicone : | 4,0% |
| D | SEPICIDE™ HB : | 0,5% |
| | SEPICIDE CI : | 03% |
| | Parfum : | 0,2% |

### MODE OPERATOIRE

préparer le mélange B + C puis ajouter A puis D.

### Exemple 30 : Soin pour les peaux grasses

### FORMULE

| | | |
|---|---|---|
| A | MICROPEARL™ M310 : | 1,0% |
| | Composition 4 : | 5,0% |
| | Isononanoate d'octyle : | 4.0% |
| B | eau : | q.s.p.100% |
| C | SEPICONTROL™ A5 : | 4,0% |
| | Parfum : | 0,1% |
| | SEPICIDE™ HB : | 0,3% |
| | SEPICIDE™ CI : | 0,2% |
| D | CAPIGEL™ 98 : | 0,5% |
| | Eau: | 10% |

### Exemple 31 : Crème aux AHA

### FORMULE

| | | |
|---|---|---|
| A | MONTANOV™ 68 : | 5,0% |
| | LIPACIDE™ PVB: | 1,05% |
| | LANOL™ 99 : | 10,0% |
| B | eau : | q.s.p.100% |
| | Acide gluconique : | 1,5% |
| | TEA (triéthanolamine) : | 0,9% |
| C | Composition 1 : | 1,5% |
| D | parfum: | 0,4% |
| | SEPICIDE™ HB: | 0,2% |
| | SEPICIDE™ Cl: | 0,4% |

### Exemple 32 : Lait solaire au monoï de Tahiti

### FORMULE

| | | |
|---|---|---|
| A | Monoï de Tahiti : | 10% |
| | LIPACIDE™ PVB : | 0,5% |
| | Composition 1 : | 2,2% |
| B | eau : | q.s.p. 100% |
| C | parfum : | 0,1% |
| | SEPICIDE™ HB : | 0,3% |
| | SEPICIDE™ Cl : | 0,1% |
| | PARSOL™ MCX : | 4,0% |

### Exemple 33 : Soin solaire pour le visage

### FORMULE

| | | |
|---|---|---|
| A | Cyclométhicone et diméthiconol : | 4,0% |
| | Composition 2 : | 3,5% |
| B | eau : | q.s.p. 100% |
| C | parfum : | 0,1% |
| | SEPICIDE™ HB : | 0,3% |
| | SEPICIDE™ Cl : | 0,21% |
| | PARSOL™ MCX : | 5,0% |
| | Micatitane : | 2,0% |
| | Acide lactique : | q.s.p. pH = 6,5 |

### Exemple 34 : Emulsion bronzante sans soleil

### FORMULE

| | | |
|---|---|---|
| A | LANOL™ 99 : | 15% |
| | MONTANOV™ 68 : | 5,0% |
| | PARSOL™ MCX : | 3,0% |
| B | eau: | q.s.p. 100% |
| | Dihydroxyacétone : | 5,0% |
| | Phosphate monosodique : | 0,2% |
| C | Composition 3 : | 0,5% |
| D | parfum : | 0,3% |
| | SEPICIDE™ HB : | 0,8% |
| | NaOH: | q.s.pH=5. |

Les caractéristiques des produits utilisés dans les exemples précédents, sont les suivantes :
Le MONTANOV™ 68 (cétéaryl glucoside, alcool cétéarylique), est une composition auto-émulsionnable telle que celles décrites dans WO 92/06778, commercialisée par la société SEPPIC.
Le MONTANOV™ 202 (arachidyl glucoside, alcool arachydilique + alcool béhènylique), est une composition auto-émulsionnable telle que celles décrites dans WO 98/17610, commercialisée par la société SEPPIC.
Le MICROPEARL™ M 305 est une poudre hydrodispersible soyeuse à base de copolymère méthylméthacrylate réticulé.
Le MICROPEARL™ M 100 est une poudre ultra fine au toucher très doux et à action matifiante commercialisée par la société MATSUMO
Le SEPICIDE™ Cl, imidazoline urée, est un agent conservateur commercialisé par la société SEPPIC.
PEMULEN™ TR est un polymère acrylique commercialisé par GOODRICH.
Le SIMULSOL™ 165 est du stéarate de glycérol auto-émulsionnable commercialisée par la société SEPPIC.
Le LANOL™ 1688 est un ester émollient à effet non gras commercialisé par la société SEPPIC.
Le LANOL™ 14M et le LANOL ™ S sont des facteurs de consistance commercialisés par la société SEPPIC.
Le SEPICIDE™ HB , qui est un mélange de phénoxyéthanol, de méthylparaben, d'éthylparaben, de propylparaben et de butylparaben, est un agent conservateur commercialisé par la société SEPPIC.
Le MONTEINE™ CA est un agent hydratant commercialisé par la société SEPPIC.
Le SCHERCEMOL™ OP est un ester émollient à effet non gras.
Le LANOL™ P est un additif à effet stabilisant commercialisé par la société SEPPIC.
Le SEPIPERL™ N est un agent nacrant, commercialisé par la société SEPPIC, à base d'un mélange d'alkyl polyglucosides tels que ceux décrits dans WO 95/13863.
Le MONTANOV™ S est un agent nacrant, commercialisé par la société SEPPIC, à base d'un mélange d'alkyl polyglucosides tels que ceux décrits dans WO 95/13863.
Le PECOSIL™ PS100 est du diméthicone copolyol phosphate commercialisé par la société PHOENIX.
Le LANOL™ 99 est de l'isononyl isononanoate commercialisé par la société SEPPIC.
Le LANOL™ 37T est du triheptanoate de glycérol, commercialisé par la société SEPPIC.
Le SEPIFEEL™ ONE est un mélange de palmitoylproline, de palmitoyl glutamate de magnésium et de palmitoyl sarcosinate de magnésium, tel que ceux décrits dans FR 2787323.
Le SOLAGUM™ L est un carraghénane commercialisé par la société SEPPIC.
Le MARCOL™ 82 est une huile de paraffine commercialisée par la société ESSO.
Le LANOL™ 84D est du malate de dioctyle commercialisé par la société SEPPIC.
Le PARSOL™ MCX est du paraméthoxycinnamate d'(éthyl hexyle) commercialisé par la société GIVAUDAN.
I' EUSOLEX™ 4360 est de la benzophénone-3 commercialisé par la société MERCK.
Le DOW CORNING™ 245 Fluid est de la cyclométhicone, commercialisée par la société DOW CORNING.
Le LIPACIDE™ PVB,est un hydrolysat de protéines de blé palmitoylé, est commercialisée par la société SEPPIC.
Le SEPICONTROL™ A5 est un mélange capryloyl glycine, sarcosine, extrait de cinnamon zylanicum, commercialisé par la société SEPPIC, tel que ceux décrits dans la demande internationale de brevet PCT/FR98/01313 déposée le 23 juin 1998.
Le CAPIGEL™ 98 est un copolymère d'acrylates commercialisé par la société SEPPIC.
Le LANOL™ 2681 est un mélange caprylate, caprate de coprah, commercialisé par la société SEPPIC.

## Revendications

1. Composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20% à 70% en poids, et de préférence de 25% à 50% en poids, d'un polyélectrolyte branché ou réticulé, **caractérisé en ce que** ledit polyélectrolyte est un copolymère à base d'au moins un monomère possédant une fonction acide fort, copolymérisé soit avec au moins un monomère possédant une fonction acide faible, soit avec au moins un monomère neutre et **caractérisé en ce que** le solvant constitutif de la phase huile est choisi parmi le squalane ou le polyisobutène hydrogéné.

2. Composition telle que définie à la revendication 1, **caractérisé en ce que** le solvant constitutif de la phase huile est le polyisobutène hydrogéné.

3. Composition telle que définie à la revendication 1, **caractérisé en ce que** le solvant constitutif de la phase huile est le squalane.

4. Composition telle que définie à l'une quelconques des revendications 1 à 3, dans laquelle le ou les agents émulsifiants du type eau dans huile, sont choisis parmi le monooléate de sorbitan, l'isostéarate de sorbitan ou l'oléate de sorbitan éthoxylé avec 5 moles d'oxyde d'éthylène.

5. Composition telle que définie à l'une quelconques des revendications 1 à 4, dans laquelle le ou les agents émulsifiants du type huile dans eau sont choisis parmi l'oléate de sorbitan éthoxylé avec 20 moles d'oxyde d'éthylène, l'huile de ricin éthoxylée à 40 moles d'oxyde d'éthylène, le laurate de sorbitan éthoxylé à 20 moles d'oxyde d'éthylène, l'alcool laurique éthoxylé à 7 moles d'oxyde d'éthylène.

6. Composition telle que définie à l'une quelconques des revendications 1 à 5, dans laquelle le ou les agents émulsifiants du type huile dans eau sont choisis parmi les composés de formule (I) :
R₁-O-[CH(R₂)-CH₂-O]ₙ-(G)ₓ-H (I)
dans laquelle R₁ représente un radical hydrocarboné linéaire ou ramifié, saturé ou insaturé comprenant de 1 à 30 atomes de carbones, R₂ représente un atome d'hydrogène ou radical alkyle comprenant 1 ou 2 atomes de carbone, G représente le reste d'un saccharide, x représente un nombre décimal compris entre 1 et 5, et n est égal, soit à zéro, soit à un nombre entier compris entre 1 et 30.

7. Composition telle que définie à la revendication 6, pour laquelle, dans la formule (I), x, est compris entre 1 et 3, plus particulièrement entre 1,05 et 2,5, tout particulièrement entre 1,1 et 2,0 et de préférence, inférieur ou égal à 1,5.

8. Composition telle que définie à l'une des revendications 6 ou 7 pour laquelle, dans la formule (I), G représente le reste du glucose ou le reste du xylose et n est égal à 0.

9. Composition telle que définie à l'une quelconque des revendications 6 à 8, pour laquelle, dans la formule (I), R₁ représente un radical comportant de 8 à 18 atomes de carbone et plus particulièrement un radical octyle, décyle, undécyle, dodécyle, tétradécyle ou hexadécyle, lesdits radicaux étant linéaires ou ramifiés.

10. Composition telle que définie à l'une quelconque des revendications 1 à 9 pour laquelle, la fonction acide fort du monomère en comportant est la fonction acide sulfonique ou la fonction acide phosphonique partiellement ou totalement salifiée et de préférence le monomère est l'acide 2-méthyl 2-[(1-oxo 2-propènyl) amino] 1-propanesulfonique partiellement ou totalement salifiée sous forme d'un sel de métal alcalin tel que par exemple le sel de sodium ou le sel de potassium, de sel d'ammonium, de sel d'un amino alcool tel que par exemple le sel de monéthanolamine ou de sel d'un amino acide tel que par exemple le sel de lysine.

11. Composition telle que définie à l'une quelconque des revendications 1 à 10 pour laquelle, la fonction acide faible du monomère en comportant est la fonction acide carboxylique et de préférence, ledit monomère est choisi parmi l'acide acrylique, l'acide méthacrylique, l'acide itaconique ou l'acide maléique partiellement ou totalement salifié.

12. Composition telle que définie à l'une quelconque des revendications 1 à 11 pour laquelle le monomère neutre est choisi parmi l'acrylate de (2-hydroxy éthyle), l'acrylate de (2,3-dihydroxy propyle), le méthacrylate de (2-hydroxy éthyle), le méthacrylate de (2,3-dihydroxy propyle), ou un dérivé éthoxylé de poids moléculaire compris entre 400 et 1000, de chacun de ces esters.

13. Composition telle que définie aux revendications 10 et 12, dans laquelle le polyélectrolyte, est un copolymère comportant en proportions molaires, entre 30% et 90% et plus particulièrement, entre 50% et 90% d'acide 2-méthyl 2-[(1-oxo 2-propènyl) amino] 1-propane-sulfonique partiellement ou totalement salifié, et entre 10% et 70% et plus particulièrement, entre 10% et 50% d'acrylate de (2-hydroxy éthyle).

14. Composition telle que définie à la revendication 13, comprenant de 20% à 60% en poids et de préférence de 25% à 45% en poids du copolymère.

15. Composition telle que définie aux revendications 13 ou 14, dans laquelle le copolymère comporte en proportions molaires de 60% à 90% de sel de sodium ou de sel d'ammonium de l'acide 2-méthyl 2-[(1-oxo 2-propènyl) amino] 1-propanesulfonique et de 1O% à 40% d'acrylate de (2-hydroxy éthyle).

16. Composition telle que définie aux revendications 10 et 11, dans laquelle le polyélectrolyte, est un copolymère comportant en proportions molaires, entre 30% et 90% et plus particulièrement, entre 30 et 45% de sel de sodium de sel d'ammonium, de sel de monoéthanolamine ou de sel de lysine de l'acide 2-méthyl 2-[(1-oxo 2-propènyl) amino] 1-propanesulfonique et entre 10% et 70% et plus particulièrement de entre 55% et 70% d'acide acrylique partiellement ou totalement salifié sous forme de sel de sodium, de sel d'ammonium, de sel de monoéthanolamine ou de sel de lysine.

17. Composition telle que définie à l'une quelconque des revendications 1 à 16, **caractérisée en ce que** le polyélectrolyte est réticulé et/ou branché avec un composé diéthylénique ou polyéthylénique dans la proportion molaire exprimée par rapport aux monomères mis en oeuvre, de 0,005% à 1%, et de préférence de 0,01 % à 0,5 % et, plus particulièrement de 0,1 % à 0,25 %.

18. Composition telle que définie à la revendication 17, **caractérisée en ce que** l'agent de réticulation et/ou de ramification est choisi parmi l'acide diallyloxyacétique ou un de ses sels comme le diallyloxyacétate de sodium, le diméthacrylate d'éthylèneglycol, le diacrylate d'éthylèneglycol, le diallyl urée, le triméthylol propanetriacrylate, le méthylène bis(acrylamide), le triallylamine ou un mélange de ces composés.

19. Composition telle que définie à l'une quelconque des revendications 1 à 18, **caractérisée qu'**elle contient de 4% à 10% en poids, d'agents émulsifiants.

20. Composition telle que définie à la revendication 19, dans laquelle de 20% à 50% et plus particulièrement de 25% à 40% du poids total des émulsifiants sont du type eau dans huile et de 80% à 50% et plus particulièrement de 75 à 60% en poids sont du type huile dans eau.

21. Composition telle que définie à l'une quelconque des revendications 1 à 20, **caractérisée en ce que** la phase huile représente de 15% à 40%, et de préférence de 20% à 25%, de son poids total.

22. Composition telle que définie à l'une quelconque des revendications 1 à 21, **caractérisée en ce qu'**elle comporte en outre, un ou plusieurs additifs choisis parmi les agents complexant, les agents de transfert ou les agents limiteurs de chaînes.

23. Composition cosmétique, dermocosmétique, dermopharmaceutique ou pharmaceutique, **caractérisée en ce qu'**elle comprend de 0,1 % à 10 % en poids, de la composition telle que définie à l'une quelconque des revendications 1 à 22.

24. Composition cosmétique, dermocosmétique, dermopharmaceutique ou pharmaceutique telle que définie à la revendication 23, sous la forme d'un lait, d'une lotion, d'un gel, d'une crème, d'un savon, d'un bain moussant, d'un baume, d'un shampooing ou d'un après shampooing.

25. Utilisation d'une composition telle que définie à l'une des revendications 1 à 22, pour préparer des compositions cosmétiques, dermocosmétiques, dermopharmaceutiques ou pharmaceutiques.

## Claims

1. Composition comprising an oil phase, an aqueous phase, at least one emulsifying agent of water-in-oil (W/O) type and at least one emulsifying agent of oil-in-water (O/W) type, in the form of a self-invertible inverse latex comprising from 20% to 70% by weight, and preferably from 25% to 50% by weight, of a branched or crosslinked polyelectrolyte, **characterized in that** said polyelectrolyte is a copolymer based on at least one monomer possessing a strong acid functional group, copolymerized either with at least one monomer possessing a weak acid functional group, or with at least one neutral monomer and **characterized in that** the constituent solvent of the oil phase is chosen from squalane and hydrogenated polyisobutene.

2. Composition as defined in Claim 1, **characterized in that** the constituent solvent of the oil phase is hydrogenated polyisobutene.

3. Composition as defined in Claim 1, **characterized in that** the constituent solvent of the oil phase is squalane.

4. Composition as defined in any one of Claims 1 to 3, in which the emulsifying agent(s) of water-in-oil type are chosen from sorbitan monooleate, sorbitan isostearate and sorbitan oleate ethoxylated with 5 mol of ethylene oxide.

5. Composition as defined in any one of Claims 1 to 4, in which the emulsifying agent(s) of oil-in-water type are chosen from sorbitan oleate ethoxylated with 20 mol of ethylene oxide, castor oil ethoxylated with 40 mol of ethylene oxide, sorbitan laurate ethoxylated with 20 mol of ethylene oxide and lauryl alcohol ethoxylated with 7 mol of ethylene oxide.

6. Composition as defined in one of Claims 1 to 5, in which the emulsifying agent(s) of oil-in-water type are chosen from compounds of formula (I):
R₁-O-[CH(R₂)-CH₂-O]ₙ-(G)ₓ-H (I)
in which R₁ represents a saturated or unsaturated, linear or branched hydrocarbon-based radical comprising from 1 to 30 carbon atoms, R₂ represents a hydrogen atom or an alkyl radical comprising 1 or 2 carbon atoms, G represents the residue of a saccharide, x represents a decimal number between 1 and 5 and n is equal either to zero or to an integer between 1 and 30.

7. Composition as defined in Claim 6, for which, in the formula (I), x is between 1 and 3, more particularly between 1.05 and 2.5, most particularly between 1.1 and 2.0 and preferably less than or equal to 1.5.

8. Composition as defined in either of Claims 6 and 7, for which, in the formula (I), G represents the glucose residue or the xylose residue and n is equal to 0.

9. Composition as defined in any one of Claims 6 to 8, for which, in the formula (I), R₁ represents a radical comprising from 8 to 18 carbon atoms and more particularly an octyl, decyl, undecyl, dodecyl, tetradecyl or hexadecyl radical, said radicals being linear or branched.

10. Composition as defined in any one of Claims 1 to 9, for which the strong acid functional group of the monomer comprising it is the sulphonic acid functional group or the phosphonic acid functional group, partially or completely salified, and preferably the monomer is 2-methyl-2-[(1-oxo-2-propenyl)amino]-1-propanesulphonic acid, partially or completely salified in the form of an alkali metal salt such as, for example, the sodium salt or the potassium salt, of the ammonium salt, of the salt of an amino alcohol such as, for example, the monoethanolamine salt or of the salt of an amino acid such as, for example, the lysine salt.

11. Composition as defined in any one of Claims 1 to 10, for which the weak acid functional group of the monomer comprising it is the carboxylic acid functional group and preferably said monomer is chosen from partially or completely salified acrylic acid, methacrylic acid, itaconic acid or maleic acid.

12. Composition as defined in any one of Claims 1 to 11, for which the neutral monomer is chosen from 2-hydroxyethyl acrylate, 2,3-dihydroxypropyl acrylate, 2-hydroxyethyl methacrylate, 2,3-dihydroxypropyl methacrylate, or an ethoxylated derivative, with a molecular weight between 400 and 1000, of each of these esters.

13. Composition as defined in Claims 10 and 12, in which the polyelectrolyte is a copolymer comprising, in molar proportions, between 30% and 90%, and more particularly between 50% and 90%, of partially or completely salified 2-methyl-2-[(1-oxo-2-propenyl)amino]-1-propanesulphonic acid and between 10% and 70%, and more particularly between 10% and 50%, of 2-hydroxyethyl acrylate.

14. Composition as defined in Claim 13, comprising from 20% to 60% by weight, and preferably from 25% to 45% by weight, of the copolymer.

15. Composition as defined in Claims 13 and 14, in which the copolymer comprises, in molar proportions, from 60% to 90% of the sodium salt or the ammonium salt of the 2-methyl-2-[(1-oxo-2-propenyl)amino]-1-propanesulphonic acid and from 10% to 40% of 2-hydroxyethyl acrylate.

16. Composition as defined in Claims 10 and 11, in which the polyelectrolyte is a copolymer comprising, in molar proportions, between 30% and 90%, and more particularly between 30 and 45%, of the sodium salt, of the ammonium salt, of the monoethanolamine salt or of the lysine salt of 2-methyl-2- [(1-oxo-2-propenyl)amino] -1-propanesulphonic acid and between 10% and 70%, and more particularly between 55% and 70%, of partially or completely salified acrylic acid in the form of the sodium salt, of the ammonium salt, of the monoethanolamine salt or of the lysine salt.

17. Composition as defined in any one of Claims 1 to 16, **characterized in that** the polyelectrolyte is crosslinked and/or branched with a diethylenic or polyethylenic compound in the molar proportion expressed relative to the monomers used, of 0.005% to 1%, and preferably from 0.01% to 0.5%, and more particularly from 0.1% to 0.25%.

18. Composition as defined in Claim 17, **characterized in that** the crosslinking and/or branching agent is chosen from diallyloxyacetic acid or one of its salts such as sodium diallyloxyacetate, ethylene glycol dimethacrylate, ethylene glycol diacrylate, diallylurea, trimethylolpropane triacrylate, methylenebis(acrylamide), triallylamine or a mixture of these compounds.

19. Composition as defined in any one of Claims 1 to 18, **characterized in that** it contains from 4% to 10% by weight of emulsifying agents.

20. Composition as defined in Claim 19, in which from 20% to 50%, and more particularly from 25% to 40% of the total weight of the emulsifiers are of water-in-oil type and from 80% to 50%, and more particular from 75 to 60% by weight are of oil-in-water type.

21. Composition as defined in any one of Claims 1 to 20, **characterized in that** the oil phase represents from 15% to 40%, and preferably from 20% to 25%, of its total weight.

22. Composition as defined in any one of Claims 1 to 21, **characterized in that** it comprises, in addition, one or more additives chosen from complexing agents, transfer agents or chain-limiting agents.

23. Cosmetic, dermocosmetic, dermopharmaceutical or pharmaceutical composition, **characterized in that** it comprises from 0.1% to 10% by weight of the composition as defined in any one of Claims 1 to 22.

24. Cosmetic, dermocosmetic, dermopharmaceutical or pharmaceutical composition, as defined in Claim 23, in the form of a milk, a lotion, a gel, a creme, a soap, a foam bath, a balm, a shampoo or a conditioner.

25. Use of a composition as defined in one of Claims 1 to 22, to prepare cosmetic, dermocosmetic, dermopharmaceutical or pharmaceutical compositions.

## Patentansprüche

1. Zusammensetzung, welche eine Ölphase, eine wässrige Phase, mindestens einen Emulgator des Typs Wasser-in-Öl (W/O) und mindestens einen Emulgator des Typs Öl-in-Wasser (O/W) umfasst und welche in Form einer milchigen Umkehremulsion, bei der die Phasenumkehr selbsttätig stattfinden kann, vorliegt und welche 20 bis 70 Gew.-%, vorzugsweise jedoch 25 bis 50 Gew.-%, eines verzweigten und/oder vernetzten Polyelektrolyten umfasst, **dadurch gekennzeichnet, dass** es sich bei dem Polyelektrolyten um ein Copolymer handelt, und zwar eines auf Basis mindestens eines Monomers, das eine starke Säuregruppe aufweist und das entweder mit mindestens einem Monomer, das eine schwache Säuregruppe aufweist, oder mit mindestens einem neutralen Monomer copolymerisiert ist, sowie **dadurch gekennzeichnet, dass** das Lösemittel, welches in der Ölphase enthalten ist, aus Squalan oder hydriertem Polyisobuten gewählt wird.

2. Zusammensetzung nach der Begriffsbestimmung in Anspruch 1, **dadurch gekennzeichnet, dass** es sich bei dem Lösemittel, welches in der Ölphase enthalten ist, um hydriertes Polyisobuten handelt.

3. Zusammensetzung nach der Begriffsbestimmung in Anspruch 1, **dadurch gekennzeichnet, dass** es sich bei dem Lösemittel, welches in der Ölphase enthalten ist, um Squalan handelt.

4. Zusammensetzung nach der Begriffsbestimmung in einem beliebigen der Ansprüche 1 bis 3, wobei der oder die Emulgatoren des Typs Wasser-in-Öl aus Sorbitanmonooleat, Sorbitanisostearat oder ethoxyliertem Sorbitanoleat mit 5 mol Ethylenoxid gewählt werden.

5. Zusammensetzung nach der Begriffsbestimmung in einem beliebigen der Ansprüche 1 bis 4, wobei der oder die Emulgatoren des Typs Wasser-in-Öl aus ethoxyliertem Sorbitanoleat mit 20 mol Ethylenoxid, ethoxyliertem Rizinusöl mit 40 mol Ethylenoxid, ethoxyliertem Sorbitanlaurat mit 20 mol Ethylenoxid, ethoxyliertem Laurinalkohol mit 7 mol Ethylenoxid gewählt werden.

6. Zusammensetzung nach der Begriffsbestimmung in einem beliebigen der Ansprüche 1 bis 5, wobei der oder die Emulgatoren des Typs Öl-in-Wasser aus den Verbindungen nach Formel (I) gewählt werden:
R₁-O-[CH(R₂)-CH₂-O]ₙ-(G)ₓ-H (I)
wobei R₁ für ein geradkettiges oder verzweigtes, gesättigtes oder ungesättigtes Wasserstoffradikal mit 1 bis 30 Kohlenstoffatomen steht und R₂ für ein Wasserstoffatom oder ein Alkylradikal mit 1 oder 2 Kohlenstoffatomen steht und G für einen Saccharidrest steht und x für eine Dezimalzahl von 1 bis 5 steht und n entweder gleich null oder gleich einer Zahl von 1 bis 30 ist.

7. Zusammensetzung nach der Begriffsbestimmung in Anspruch 6, bei welcher die Zahl x in der Formel (I) zwischen 1 und 3, insbesondere zwischen 1,05 und 2,5, sowie ganz besonders zwischen 1,1 und 2,0 liegt und vorzugsweise kleiner oder gleich 1,5 ist.

8. Zusammensetzung nach der Begriffsbestimmung in einem der Ansprüche 6 oder 7, bei welcher G in der Formel (I) für einen Glucoserest oder einen Xyloserest steht und n gleich 0 ist.

9. Zusammensetzung nach der Begriffsbestimmung in einem beliebigen der Ansprüche 6 bis 8, bei welcher R₁ in der Formel (I) für ein Radikal mit 8 bis 18 Kohlenstoffatomen und insbesondere für ein Octyl-, Decyl-, Undecyl-, Dodecyl-Tetradecyl- oder Hexadecylradikal steht, wobei die Radikale geradkettig oder verzweigt sein können.

10. Zusammensetzung nach der Begriffsbestimmung in einem beliebigen der Ansprüche 1 bis 9, bei welcher es sich bei der starken funktionellen Säuregruppe, welche das Monomer aufweist, um eine teilweise oder vollständig in die Salzform überführte funktionelle Sulfonsäuregruppe oder funktionelle Phosphonsäuregruppe handelt, wobei es sich bei dem Monomer vorzugsweise um teilweise oder vollständig in die Salzform überführte 2-Methyl-2-[(1-oxo-2-propenyl)amino]-1-propansulfonsäure handelt, welche in Form eines Alkalimetallsalzes wie beispielsweise des Natriumsalzes oder des Kaliumsalzes, in Form eines Ammoniumsalzes, in Form eines Aminoalkoholsalzes wie beispielsweise des Monoethanolaminsalzes oder in Form eines Aminosäuresalzes wie beispielsweise des Lysinsalzes vorliegt.

11. Zusammensetzung nach der Begriffsbestimmung in einem beliebigen der Ansprüche 1 bis 10, bei welcher es sich bei der schwachen funktionellen Säuregruppe, welche das Monomer aufweist, um eine funktionelle Carbonsäuregruppe handelt, wobei das Monomer vorzugsweise aus Acrylsäure, Methacrylsäure, Itaconsäure oder Maleinsäure, teilweise oder vollständig in die Salzform überführt, gewählt wird.

12. Zusammensetzung nach der Begriffsbestimmung in einem beliebigen der Ansprüche 1 bis 11, bei welcher das neutrale Monomer aus (2-Hydroxyethyl)acrylat, (2,3-Dihydroxypropyl)acrylat, (2-Hydroxyethyl)methacrylat, (2,3-Dihydroxypropyl)-methacrylat oder einem ethoxylierten Derivat eines dieser Ester, welches eine Molekülmasse zwischen 400 und 1000 aufweist, ausgewählt wird.

13. Zusammensetzung nach der Begriffsbestimmung in den Ansprüchen 10 und 12, bei welcher es sich bei dem Polyelektrolyt um ein Copolymer handelt, welches, in molaren Anteilen ausgedrückt, zwischen 30 % und 90 %, insbesondere jedoch zwischen 50 % und 90 %, an teilweise oder vollständig in die Salzform überführter 2-Methyl-2-[(1-oxo-2-propenyl)amino]-1-propansulfonsäure sowie zwischen 10 % und 70 %, insbesondere jedoch zwischen 10 % und 50 %, an (2-Hydroxyethyl)acrylat umfasst.

14. Zusammensetzung nach der Begriffsbestimmung in Anspruch 13, welche zwischen 20 und 60 Gew.-%, vorzugsweise jedoch zwischen 25 und 45 Gew.-%, des Copolymers umfasst.

15. Zusammensetzung nach der Begriffsbestimmung in den Ansprüchen 13 und 14, bei welcher das Copolymer, in molaren Anteilen ausgedrückt, 60 % bis 90 % an Natriumsalz oder Ammoniumsalz der 2-Methyl-2-[(1-oxo-2-propenyl)amino]-1-propansulfonsäure sowie 10 % bis 40 % an (2-Hydroxyethyl)acrylat umfasst.

16. Zusammensetzung nach der Begriffsbestimmung in den Ansprüchen 10 und 11, bei welcher es sich bei dem Polyelektrolyt um ein Copolymer handelt, welches, in molaren Anteilen ausgedrückt, zwischen 30 % und 90 %, insbesondere jedoch zwischen 30 % und 45 %, an Natriumsalz, an Ammoniumsalz, an Monoethanolaminsalz oder Lysinsalz der 2-Methyl-2-[(1-oxo-2-propenyl)amino]-1-propansulfonsäure sowie zwischen 10 % und 70 %, insbesondere jedoch zwischen 55 % und 70 %, an teilweise oder vollständig in die Salzform überführter Acrylsäure in Form des Natriumsalzes, des Ammoniumsalzes, des Monoethanolaminsalzes oder des Lysinsalzes umfasst.

17. Zusammensetzung nach der Begriffsbestimmung in einem beliebigen der Ansprüche 1 bis 16, **dadurch gekennzeichnet, dass** das Polyelektrolyt vernetzt und/oder verzweigt ist und eine diethylenische oder polyethylenische Verbindung aufweist, deren molarer Anteil bezogen auf die eingesetzten Monomere zwischen 0,005 % und 1 %, vorzugsweise jedoch zwischen 0,01 % und 0,5 %, und insbesondere zwischen 0,1 % und 0,25 % beträgt.

18. Zusammensetzung nach der Begriffsbestimmung in Anspruch 17, **dadurch gekennzeichnet, dass** das Vernetzungs- und/oder Verzweigungsmittel aus Diallyloxyessigsäure oder einem ihrer Salze wie etwa Natriumdiallyloxyacetat, aus Ethylenglykoldimethacrylat, aus Ethylenglykoldiacrylat, aus Diallylharnstoff, aus Trimethylolpropantriacrylat, aus Methylen-bis(acrylamid), aus Triallylamin oder aus einer Mischung dieser Verbindungen gewählt wird.

19. Zusammensetzung nach der Begriffsbestimmung in einem beliebigen der Ansprüche 1 bis 18, **dadurch gekennzeichnet, dass** sie 4 bis 10 Gew.-% an Emulgatoren enthält.

20. Zusammensetzung nach der Begriffsbestimmung in Anspruch 19, wobei es sich bei 20 % bis 50 % und insbesondere bei 25 % bis 40 % des Gesamtgewichtes der Emulgatoren um solche des Typs Wasser-in-Öl und bei 80 % bis 50 % und insbesondere 75 bis 60 Gew.-% um solche des Typs Öl-in-Wasser handelt.

21. Zusammensetzung nach der Begriffsbestimmung in einem beliebigen der Ansprüche 1 bis 20, **dadurch gekennzeichnet, dass** die Ölphase 15 % bis 40 % und vorzugsweise 20 % bis 25 % ihres Gesamtgewichtes ausmacht.

22. Zusammensetzung nach der Begriffsbestimmung in einem beliebigen der Ansprüche 1 bis 21, **dadurch gekennzeichnet, dass** sie weiterhin einen oder mehrere Zusatzstoffe umfasst, welche aus den Komplexierungsmitteln, den Übertragungsmitteln oder den Mitteln zur Begrenzung der Kettenlänge gewählt werden.

23. Kosmetische, hautkosmetische, hautpharmazeutische oder pharmazeutisch Zusammensetzung, **dadurch gekennzeichnet, dass** sie 0,1 bis 10 Gew.-% der Zusammensetzung nach der Begriffsbestimmung in einem beliebigen der Ansprüche 1 bis 22 umfasst.

24. Kosmetische, hautkosmetische, hautpharmazeutische oder pharmazeutische Zusammensetzung nach der Begriffsbestimmung in Anspruch 23, welche in Form einer Milch, einer Lotion, eines Gels, einer Creme, einer Seife, eines Schaumbads, eines Balsams, eines Shampoos oder eines Nachbehandlungsmittels für die Haarwäsche vorliegt.

25. Verwendung einer Zusammensetzung gemäß der Begriffsbestimmung in einem der Ansprüche 1 bis 22, um kosmetische, hautkosmetische, hautpharmazeutische oder pharmazeutische Zusammensetzungen herzustellen.
